# FASCICULE DE BREVET EUROPEEN

(11) **EP 2 736 652 B1**
(45) Date de publication et mention de la délivrance du brevet: **10.08.2016**
(21) Numéro de dépôt: 12773008.3
(22) Date de dépôt: 07.06.2012
(51) Int. Cl.: B05B 11/00, B05B 15/02, B65D 83/34, B08B 7/00, B65D 83/40

(54) **TETE DE DISTRIBUTION DE PRODUIT FLUIDE**
AUSGABEKOPF FÜR EIN FLÜSSIGES PRODUKT
FLUID PRODUCT DISPENSING HEAD

(30) Priorité: 25.07.2011 FR 1156777
(43) Date de publication de la demande: 04.06.2014
(73) Titulaire: Aptar France SAS, 27110 Le Neubourg (FR)
(72) Inventeur: DUQUET, Frédéric, F-78121 Crespières (FR)
(74) Mandataire: CAPRI
(86) Numéro de dépôt international: PCT/FR2012/051275
(87) Numéro de publication internationale: WO 2013/014345

(56) Documents cités:
- EP-A1- 0 882 585
- EP-A2- 0 461 895
- FR-A1- 2 924 484
- US-A- 6 067 908
- US-A1- 2005 056 663

## Description

La présente invention concerne une tête de distribution de produit fluide destinée à être associée à un organe de distribution, tel qu'une pompe, et à un réservoir pour former ensemble un distributeur de produit fluide. La tête comprend un orifice de distribution de produit fluide au niveau duquel un utilisateur peut recueillir le produit fluide distribué. La tête comprend également un capot de protection amovible qui masque l'orifice de distribution en condition de stockage, le capot devant être retiré pour recueillir le produit fluide distribué à travers l'orifice de distribution. L'invention concerne également un distributeur comprenant un réservoir, un organe de distribution et une tête de distribution de l'invention. Les domaines d'application privilégiés de la présente invention sont ceux de la cosmétique, de la pharmacie ou encore de la parfumerie.

Des têtes de distribution de ce type sont très fréquemment utilisées dans le domaine de la cosmétique pour la distribution de produits visqueux, tels que des crèmes, des gels, etc. Le produit fluide sort de l'orifice de distribution sous la forme d'un cordon ou d'une noisette. L'utilisateur peut recueillir le produit fluide à l'aide d'un doigt ou sur une autre surface d'application souhaitée. Dans le cas de la présente invention, l'orifice de distribution désigne tout type d'ouverture terminale au niveau de laquelle le produit fluide est accessible par l'utilisateur. En général, l'orifice de distribution est formé au niveau d'un poussoir qui est déplaçable axialement en va-et-vient par l'utilisateur à l'aide d'un ou de plusieurs doigt(s). L'orifice de distribution s'étend souvent latéralement ou radialement par rapport à l'axe de déplacement du poussoir. Ainsi, lorsque l'on appuie sur le poussoir, l'orifice de distribution se déplace axialement sur une distance correspondant à la course du poussoir. Afin d'empêcher tout actionnement involontaire ou intempestif du poussoir, et de protéger l'orifice de distribution, le poussoir est conventionnellement coiffé ou masqué par un capot de protection qui se présente en général sous la forme d'un godet renversé. Le capot comprend une paroi supérieure disposée au-dessus du poussoir et une paroi latérale souvent cylindrique qui entoure le poussoir. Le capot peut être maintenu en place sur le distributeur au niveau de son bord annulaire inférieur, qui peut par être encliqueté sur un élément fixe du distributeur, qui peut être le réservoir ou une bague de fixation. De ce fait, l'orifice de distribution fait face à la paroi latérale du capot. Le distributeur est alors dans sa condition de stockage, étant donné qu'il n'est pas possible d'accéder au poussoir masqué par le capot. Lorsque l'utilisateur veut se servir du distributeur, il commence par retirer axialement le capot de manière à démasquer le poussoir qui est alors accessible. L'utilisateur peut alors appuyer sur le poussoir de manière à le déplacer axialement, ce qui engendre la distribution de produit fluide à travers l'orifice de distribution. Bien entendu, l'utilisateur va chercher à recueillir la totalité du produit fluide distribué au niveau de l'orifice de distribution. Toutefois, il subsiste toujours un résidu de produit fluide dans l'orifice de distribution et/ou autour de l'orifice de distribution. Etant donné qu'il est extrêmement difficile de recueillir ce résidu de produit fluide, l'utilisateur laisse le distributeur dans cet état et remet le capot en place. De ce fait, ce résidu de produit fluide va dessécher et se détériorer, engendrant l'apparition de micro-organismes, germes, bactéries, microbes, etc. A la prochaine distribution, l'utilisateur va à nouveau retirer le capot et appuyer sur le poussoir de manière à distribuer du produit fluide à travers l'orifice de distribution. La nouvelle dose de produit fluide va bien entendu entrer en contact avec le résidu desséché et détérioré de produit fluide provenant de la distribution précédente. Il s'ensuit que la nouvelle dose de produit fluide va elle aussi être contaminée par les micro-organismes, germes, bactéries, microbes, etc. provenant du résidu de produit fluide. L'hygiène du produit fluide distribué n'est donc pas assurée.

La présente invention a pour but de remédier à l'inconvénient précité de l'art antérieur en définissant une tête de distribution et un distributeur dont les doses successives distribuées ne sont pas contaminées par des résidus de produit fluide provenant de distributions précédentes. Un autre but de la présente invention est de ne pas bouleverser fondamentalement l'architecture de la tête de distribution constituée de l'orifice de distribution et du capot. Encore un autre but de l'invention est de stériliser ou de neutraliser les résidus de produit fluide sans manipulation autre que celles conventionnelles de retrait et de mise en place du capot sur l'orifice de distribution.

Le document US2005056663 A1 décrit une tête de distribution selon le préambule de la revendication 1.

Pour atteindre ces différents buts, la présente invention propose une tête de distribution de produit fluide selon la revendication 1. En d'autres termes, on se sert du capot comme support d'un rayonnement dirigé vers l'orifice de distribution éventuellement souillé par un résidu de produit fluide. Avantageusement, le capot comprend une source de rayonnement émettant le rayonnement vers l'orifice de distribution. En variante, la source de rayonnement peut également être déportée dans un autre élément constitutif du distributeur.

Selon une autre caractéristique intéressante de l'invention, la source de rayonnement est disposée à proximité directe de l'orifice de distribution. De préférence, la source de rayonnement est disposée juste en face de l'orifice de distribution. Ainsi, un maximum de puissance émis par le rayonnement est focalisé sur l'orifice de distribution. Le rayonnement peut atteindre les résidus de produit fluide situés autour de l'orifice de distribution, mais également le produit fluide à l'intérieur de l'orifice de distribution.

Selon l'invention, le capot est déplaçable selon une direction axiale, l'orifice de distribution étant orienté transversalement à cette direction axiale. Il s'agit là d'une configuration classique, dans laquelle le capot est retiré selon la même direction axiale que le déplacement en va-et-vient du poussoir. Selon l'invention, le capot est orienté angulairement par rapport à l'orifice de distribution pour imposer le positionnement du rayonnement en face de l'orifice de distribution. On garantit ainsi que le rayonnement est toujours disposé en face de l'orifice de distribution, puisque l'utilisateur n'a pas d'autre choix de positionnement du capot.

Selon une autre forme de réalisation pratique, l'orifice de distribution est formé par un poussoir qui est déplaçable en va-et-vient selon une direction axiale. Là encore, il s'agit d'une configuration classique dans laquelle l'orifice de distribution se déplace de manière solidaire avec le poussoir. L'orifice de distribution est généralement disposé de manière transversale ou radiale, mais on peut également envisager de disposer l'orifice de distribution dans la direction axiale de déplacement du poussoir.

Selon un autre aspect intéressant, le capot intègre des moyens de déclenchement du rayonnement qui sont sensibles à mise en place du capot sur l'orifice de distribution. Avantageusement, les moyens de déclenchement comprennent un détecteur de présence à l'intérieur du capot. Le détecteur de présence peut fonctionner avec ou sans contact. D'autre part, le capot peut intégrer des moyens de temporisation aptes à couper le rayonnement au bout d'un laps de temps déterminé. Ainsi, lorsque le capot est mis en place autour de l'orifice de distribution, les moyens de déclenchement déclenchent l'émission du rayonnement vers l'orifice de distribution pendant une durée de temps qui est déterminée par les moyens de temporisation. Ainsi, l'utilisateur n'a même pas besoin de se soucier de l'activation et de la désactivation du rayonnement permettant d'irradier les éventuels résidus de produit fluide situés au niveau de l'orifice de distribution. La gestuelle classique reste inchangée.

Selon une forme de réalisation avantageuse, le rayonnement est un rayonnement Ultra-violet, avantageusement émis par une diode électroluminescente LED, ayant une longueur d'onde de l'ordre de 253-254 nanomètres, pour décontaminer ou stériliser l'éventuel résidu de produit fluide au niveau de l'orifice de distribution. En utilisant un autre type de rayonnement, il est possible d'agir sur les résidus de produit fluide ou même sur la dose de produit fluide distribuée dans un but différent.

L'invention concerne également un distributeur de produit fluide comprenant un réservoir de produit fluide, un organe de distribution, telle qu'une pompe, montée sur le réservoir, et une tête de distribution selon l'invention, montée sur l'organe de distribution, l'orifice étant formé par un poussoir déplaçable en va-et-vient selon une direction axiale, l'orifice étant orienté transversalement à la direction axiale de déplacement du poussoir, le capot étant monté sur une partie fixe du distributeur en masquant le poussoir. Avantageusement, le poussoir est bloqué en rotation, le capot étant indexé en rotation pour imposer le positionnement du rayonnement en face de l'orifice de distribution, lors de la mise en place du capot sur le poussoir.

Un des principes de la présente invention est de se servir du capot de protection coiffant l'orifice de distribution comme organe de support ou de transmission d'un rayonnement qui va stériliser ou décontaminer les éventuels résidus de produit fluide situés au niveau de l'orifice de distribution. L'indexation du capot par rapport à l'orifice de distribution permet d'imposer le positionnement correct du rayonnement par rapport à l'orifice de distribution.

L'invention sera maintenant plus amplement décrite en référence aux dessins joints donnant à titre d'exemple non limitatif un mode de réalisation de l'invention.

Sur les figures :
La figure 1 est une vue explosée en perspective d'un distributeur selon une forme de réalisation non limitative de l'invention,
La figure 2 est une vue en coupe transversale verticale à travers le distributeur de la figure 1, et
La figure 3 est une vue en coupe transversale horizontale à travers le distributeur des figures 1 et 2 au niveau de l'orifice de distribution.

Le distributeur de produit fluide représenté sur les figures est plus particulièrement destiné au produit fluide visqueux, comme des crèmes, des gels, etc. Il comprend essentiellement cinq éléments constitutifs, à savoir un réservoir 1, un organe de distribution 2 qui est une pompe, une bague de fixation 3 pour fixer la pompe sur le réservoir, un poussoir 4 monté sur la pompe et un capot 5 qui incorpore la présente invention ; a l'exception du capot 5 et dans une moindre mesure la bague de fixation 3, les autres éléments constitutifs, à savoir le réservoir 1, la pompe 2 et le poussoir 4 peuvent être de conception tout à fait conventionnelle.

Le réservoir de produit fluide 1 peut être de toutes natures, de toutes formes et réalisé en n'importe quel matériau approprié. Il définit un volume interne de stockage de produit fluide qui peut être constant ou variable. Dans le domaine de la cosmétique, il est plutôt d'usage d'utiliser des réservoirs de capacité variable, afin que le produit fluide stocké à l'intérieur ne rentre pas en contact avec l'air extérieur. Le réservoir comprend bien entendu une ouverture qui fait communiquer son volume interne avec l'extérieur.

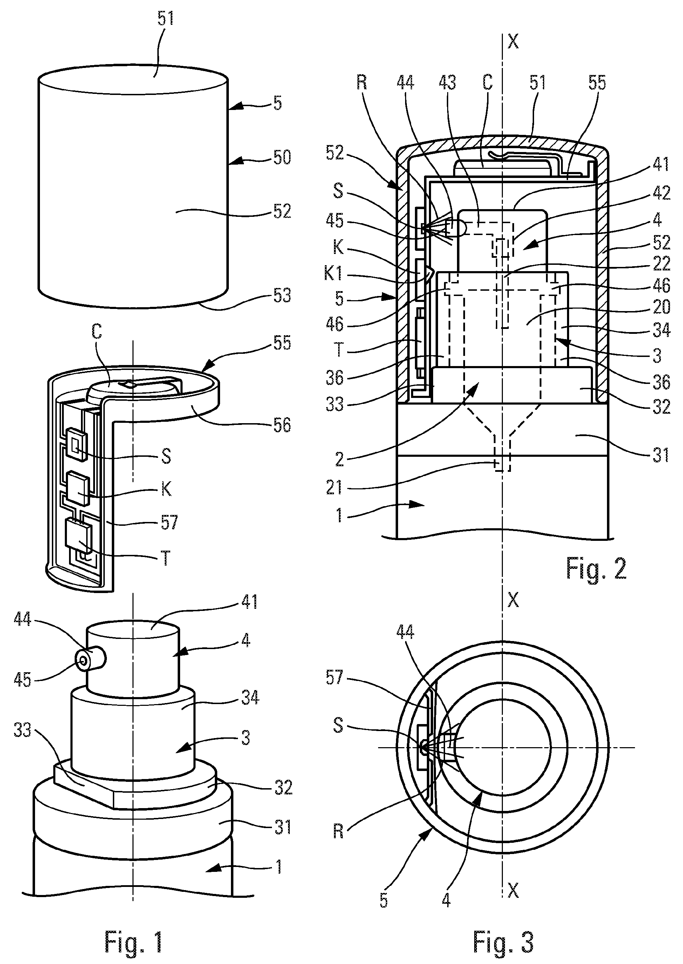

L'organe de distribution 2, comme précité, est une pompe manuelle comprenant un corps de pompe 20 définissant une entrée 21 en communication avec le réservoir 1. La pompe 2 comprend également une tige d'actionnement 22 qui est déplaçable axialement en va-et-vient à l'intérieur du corps 20 de manière à faire varier le volume d'une chambre de pompe pour mettre une dose de produit fluide sous pression. Sur la figure 2, on peut voir que la tige d'actionnement 22 s'étend le long de la direction axiale X, qui constitue également un axe de symétrie, voire de révolution, pour le distributeur. Bien que non représentée, la tige d'actionnement 22 est pourvue d'un piston adapté à coulisser de manière étanche à l'intérieur d'un fût de coulissement de la chambre de pompe. Le fonctionnement de cette pompe est tout à fait conventionnel : en enfonçant la tige d'actionnement 22 dans le corps de pompe 20, le produit fluide contenu dans la chambre de pompe est mis sous pression de manière à être refoulé à travers la tige d'actionnement. Lorsque l'on relâche la tige d'actionnement 22 qui regagne sa position de repos sous l'action d'un ressort de rappel, du produit fluide en provenance du réservoir est aspiré dans la chambre de pompe à travers l'entrée 21.

La bague de fixation 3 a pour fonction principale de maintenir la pompe 2 par rapport au réservoir 1. La fixation est de préférence définitive et étanche. La bague de fixation 3 comprend une section basse 31 en prise avec l'ouverture du réservoir 1, une section intermédiaire 32 et une section supérieure 34. On peut remarquer sur la figure 1 que la section intermédiaire 32 comporte un méplat 33 qui rompt la circularité de la géométrie. Ce méplat 33, comme on le verra ci-après, va servir à l'indexation du capot 5 par rapport au reste du distributeur. La pompe 2 est maintenue par n'importe quel moyen approprié de manière fixe et étanche à l'intérieur de la bague de fixation 3. On peut remarquer sur la figure 1 que le corps de pompe s'étend à travers les trois sections de la bague de fixation. La tige d'actionnement 22 peut faire saillie axialement vers le haut hors de la bague de fixation 3.

Le poussoir 4 est monté sur l'extrémité libre de la tige d'actionnement 22, et peut être déplacé en va-et-vient selon la direction axiale X. De cette manière, le poussoir 4 entraîne la tige d'actionnement 22 dans le corps de pompe 20. Le poussoir 4 comprend un manchon de raccordement 42 en prise autour de l'extrémité libre de la tige d'actionnement 22, ce manchon communiquant avec un embout 44 par un conduit d'alimentation interne 43. L'embout 44 forme un orifice de distribution 45 visible sur la figure 1. On peut remarquer que l'embout 44 fait saillie radialement ou latéralement vers l'extérieur, de sorte que l'orifice de distribution 45 est orienté transversalement, et plus particulièrement perpendiculairement, par rapport à la direction axiale X. Ainsi, lorsque l'on déplace le poussoir 4 axialement en va-et-vient, l'orifice de distribution 45 se déplace également de manière solidaire avec le poussoir 4. Lors de son déplacement, le poussoir 4 pénètre partiellement à l'intérieur de la section supérieure 34 de la bague de fixation 3.

Le capot de protection 5 comprend un corps de capot 50 qui est de préférence opaque. Ce corps de capot 50 présente une configuration générale en forme de godet renversé, définissant ainsi une paroi supérieure 51 et une paroi latérale sensiblement cylindrique 52 qui définit un bord inférieur annulaire 53. Une fois en place sur le distributeur, comme représenté sur la figure 2, la paroi supérieure 51 est disposée au-dessus du poussoir 4 et la paroi latérale 52 s'étend autour du poussoir 4 et de la bague de fixation 3. Plus précisément, la paroi latérale 52 entoure la section intermédiaire 32 et la section supérieure 34 de la bague de fixation : le bord annulaire inférieur 53 du capot 5 venant en appui sur la section inférieure 31 de la bague de fixation 3. On peut prévoir un encliquetage entre le capot 5 et la bague de fixation 3 pour stabiliser le maintien du capot 5 sur le distributeur en condition de stockage.

Le distributeur qui vient d'être décrit présente une conception tout à fait conventionnelle dans les domaines de la cosmétique, de la parfumerie ou encore de la pharmacie. Le poussoir 4, et plus particulièrement son orifice de distribution 45, associé au capot 5 constituent une tête de distribution de produit fluide au sens le plus large. Sans sortir du cadre de l'invention, il est possible de dissocier l'orifice de distribution du poussoir 4, par exemple de manière à rendre l'orifice fixe par rapport au réservoir 1. L'orifice de distribution 45 peut alors être relié au poussoir 4 par un conduit souple. D'autres configurations de distributeurs permettent également de dissocier l'orifice de distribution du poussoir 4. Dans le cadre de la présente invention, la tête de distribution doit être comprise comme l'association d'un orifice de distribution avec un capot de protection. Dans le cas particulier non limitatif des figures, la tête de distribution est constituée par le poussoir 4 et le capot 5.

Selon l'invention, le capot de protection 5 comprend un élément de support 55 qui est disposé à l'intérieur de l'espace formé par le capot. L'élément de support 55 peut par exemple former un plateau supérieur 56 et une languette latérale 57 reliés ensemble au niveau d'un bord du plateau de sorte que la languette 57 s'étend axialement vers le bas. Ainsi, cet élément de support 55 peut être inséré à l'intérieur du corps de capot 50 de sorte que le plateau 56 vienne se positionner juste sous la paroi supérieure 51 et la languette 57 juste contre la paroi latérale 52. Ceci est clairement visible sur la figure 2, et également compréhensible à partir de la figure 1. L'extrémité inférieure de la languette 57 s'étend sensiblement jusqu'au niveau de l'extrémité inférieure de la paroi latérale 52 du capot 5 de manière à pouvoir se positionner contre le méplat 33 de la section intermédiaire 32 de la bague de fixation 3. Ainsi, l'élément de support 55 impose l'orientation angulaire du capot de protection 5 par rapport à la bague de fixation 3, et de ce fait par rapport au restant du distributeur. Le capot 5 est donc toujours orienté de la même façon par rapport au distributeur. D'autre part, le poussoir 4 est bloqué en rotation par rapport au restant du distributeur de sorte que l'embout 44 et son orifice de distribution 45 sont toujours orientés de la même manière. Pour réaliser le blocage du poussoir 4, on peut par exemple prévoir deux ergots de guidage 46 au niveau du poussoir 4 qui coulissent dans deux rainures axiales 36 formées par la section supérieure 34 de la bague de fixation 3. Il s'agit là d'une caractéristique conventionnelle fréquemment utilisée pour bloquer le poussoir en rotation. Ainsi, le capot 5 est indexé par rapport au distributeur et l'orifice de distribution 45 est bloqué en rotation, ce qui impose le positionnement relatif de l'orifice de distribution 45 et du capot 5. Comme on peut le voir sur les figures 2 et 3, l'embout de distribution 44 est orienté vers la languette 57 dont l'extrémité inférieure vient en contact du méplat 33. Cela constitue le seul positionnement possible du capot 5 par rapport au distributeur et l'orifice de distribution.

Selon l'invention, l'élément de support 55 supporte une source de rayonnement S apte à émettre un rayonnement R destiné à irradier l'orifice de distribution 45 et son environnement proche. De cette manière, si du produit fluide résiduel subsiste au niveau de l'orifice de distribution et/ou autour de l'orifice de distribution, il sera irradié par le rayonnement R. On peut voir sur les figures 2 et 3 que la source S et son rayonnement R sont disposés à proximité, et juste en face, de l'orifice de distribution 45. La source S est disposée sur une plaque de circuit imprimée montée sur la languette 57. Pour l'activation de la source S, il est prévu des moyens de déclenchement K par exemple sous la forme d'un interrupteur comprenant un organe de déclenchement K1 qui peut être mécanique et/ou électronique. On peut par exemple prévoir que l'organe de déclenchement K1 vienne en contact avec la section supérieure 34 de la bague de fixation 3. On peut également prévoir l'organe de déclenchement K1 sous la forme d'un détecteur de présence qui détecte la présence d'un objet à l'intérieur du capot. L'organe de déclenchement K1 peut ainsi fonctionner avec ou sans contact direct. Les moyens de déclenchement K sont avantageusement associés à des moyens de temporisation T aptes à couper le rayonnement R au bout d'un laps de temps déterminé. Les moyens de temporisation peuvent par exemple agir sur les moyens de déclenchement K pour les réinitialiser. On peut par exemple prévoir une durée de rayonnement de l'ordre de 10 secondes à 1 minute. Et pour l'alimentation électrique du système, il est prévu une batterie C qui peut être disposée au niveau du plateau 56. Une fois l'élément de support 55 ainsi équipé, il est inséré dans le corps de capot 50 avec tous les éléments électroniques disposés entre l'élément de support 55 et le corps de capot, de sorte qu'aucun élément électronique n'est visible.

La manipulation de ce capot de protection 5 est strictement le même qu'un capot de protection classique. Il est mis en place et retiré en le déplaçant de manière axiale dans la direction X. Lorsque l'on le met en place, l'organe de déclenchement K1 détecte la présence du poussoir 4 et/ou de la bague de fixation 3, ou en variante vient en contact direct avec le poussoir 4 et/ou la bague de fixation 3, de manière à déclencher le rayonnement R de la source S. Au cours de cette opération, l'utilisateur n'intervient aucunement sur le déclenchement et le fonctionnement de la source S. Le rayonnement R est ainsi émis pendant un laps de temps déterminé par les moyens de temporisation T. Au terme de ce laps de temps, le rayonnement R est arrêté. Le capot de protection 5 remplit alors à nouveau une simple fonction classique de protection. Lorsque l'utilisateur retire axialement le capot 5, la source S reste inactive : ce n'est que lors de la remise en place du capot sur le distributeur que la source va à nouveau émettre son rayonnement pendant un laps de temps déterminé. Par conséquent, la gestuelle de déplacement du capot est tout à fait conventionnelle. L'utilisateur peut même ignorer la présence de la source de rayonnement et de ces composants électroniques associés.

Selon un mode d'application de la présente invention, le rayonnement R est un rayonnement Ultra-violet ayant une longueur d'onde de l'ordre de 253-254 nanomètres, apte à remplir une fonction de décontamination ou de stérilisation. Le rayonnement peut par exemple être émis par une diode électroluminescente LED. Avec une intensité de 6 à 40 milli joules par cm², on assure la destruction de 99,99% de la majorité des bactéries qui pourraient se développer au niveau de résidus de produit fluide dans la zone de l'orifice de distribution 45. L'intensité est bien entendu dépendante de la puissance de la source S, de la distance source/orifice et du temps d'irradiation du rayonnement R. Les résidus de produit fluide qui s'accumulent au niveau de l'orifice de distribution, et/ou autour de l'embout de distribution 44, sont ainsi décontaminés et/ou stérilisés, de sorte que la dose de produit fluide distribuée ultérieurement ne sera pas contaminée par les résidus de produit fluide provenant des distributions précédentes.

Dans le mode de réalisation qui vient d'être décrit, la source de rayonnement S est embarquée dans le capot 5. En variante non représentée, on peut également envisager de déporter cette source dans un autre élément constitutif du distributeur, comme par exemple la bague de fixation 3 et d'acheminer le rayonnement au moyen d'un guide d'onde ou d'une fibre optique au niveau de l'orifice de distribution. Il en est de même pour les éléments électroniques associés comme les moyens de déclenchement K et les moyens de temporisation T, ainsi que pour la batterie C, qui pourraient être logés à l'intérieur de la bague de fixation 3. Un principe de l'invention est de se servir du capot 5 comme support d'un rayonnement émis directement sur l'orifice de distribution et son environnement proche.

Selon une autre caractéristique optionnelle de l'invention, la surface de la tête autour de l'orifice de distribution comprend un photocalatyseur bactéricide, le rayonnement irradiant le photocalatyseur bactéricide. Ainsi, le rayonnement a pour fonction d'activer ou de stimuler l'effet ou les propriétés bactéricides d'une substance photosensible qui va à son tour agir sur le produit fluide présent sur les surfaces la tête pour les stériliser ou décontaminer. La surface peut s'étendre à toute la tête et même au capot. Le rayonnement, avantageusement émis par une diode électroluminescente LED, a une longueur d'onde de l'ordre de 280 à 380 nanomètres, pour activer l'effet bactéricide du photocatalyseur. Le photocalatyseur bactéricide peut être du dioxyde de titane TiO2. Le photocalatyseur bactéricide est appliqué sur la surface ou est intégré dans une paroi formant la surface.

## Revendications

1. Tête de distribution de produit fluide destinée à être associée à un organe de distribution (2), tel qu'une pompe, et à un réservoir (1) pour former ensemble un distributeur de produit fluide, la tête comprenant un orifice de distribution de produit fluide (45) par lequel le produit fluide sort de la tête de manière à être accessible pour un utilisateur, la tête comprenant également un capot de protection amovible (5) qui masque l'orifice de distribution (45) en condition de stockage, le capot (5) émettant un rayonnement (R) apte à irradier un éventuel résidu de produit fluide au niveau de l'orifice de distribution (45), **caractérisée en ce que** :
- le capot (5) est déplaçable selon une direction axiale (X), l'orifice de distribution (45) étant orienté transversalement à cette direction axiale (X), et,
- le capot (5) est orienté angulairement par rapport à l'orifice de distribution (45) pour imposer le positionnement du rayonnement (R) en face de l'orifice de distribution.

2. Tête de distribution selon la revendication 1, dans laquelle le capot (5) comprend une source de rayonnement (S) émettant le rayonnement (R) vers l'orifice de distribution (45).

3. Tête de distribution selon la revendication 2, dans laquelle la source de rayonnement (S) est disposée à proximité directe de l'orifice de distribution (45).

4. Tête de distribution selon la revendication 2 ou 3, dans laquelle la source de rayonnement (S) est disposée juste en face de l'orifice de distribution (45).

5. Tête de distribution selon l'une quelconque des revendications précédentes, dans laquelle l'orifice de distribution (45) est formé par un poussoir (4) qui est déplaçable en va-et-vient selon une direction axiale (X).

6. Tête de distribution selon l'une quelconque des revendications précédentes, dans laquelle le capot (45) intègre des moyens de déclenchement (K) du rayonnement (R) qui sont sensibles à mise en place du capot sur l'orifice de distribution (45).

7. Tête de distribution selon la revendication 6, dans laquelle les moyens de déclenchement (K) comprennent un détecteur de présence à l'intérieur du capot.

8. Tête de distribution selon l'une quelconque des revendications précédentes, dans laquelle le capot (45) intègre des moyens de temporisation (T) aptes à couper le rayonnement (R) au bout d'un laps de temps déterminé.

9. Tête de distribution selon l'une quelconque des revendications précédentes, dans laquelle le rayonnement (R) est un rayonnement Ultra-violet, avantageusement émis par une diode électroluminescente LED, ayant une longueur d'onde de l'ordre de 253-254 nanomètres, pour décontaminer ou stériliser l'éventuel résidu de produit fluide au niveau de l'orifice de distribution (45).

10. Distributeur de produit fluide comprenant :
- un réservoir de produit fluide (1),
- un organe de distribution (2), telle qu'une pompe, montée sur le réservoir (1), et
- une tête de distribution selon l'une quelconque des revendications précédentes, montée sur l'organe de distribution (2),
l'orifice (45) étant formé par un poussoir (4) déplaçable en va-et-vient selon une direction axiale (X), l'orifice (45) étant orienté transversalement à la direction axiale (X) de déplacement du poussoir (4), le capot (5) étant monté sur une partie fixe (3) du distributeur en masquant le poussoir (4).

11. Distributeur selon la revendication 12, dans lequel le poussoir (4) est bloqué en rotation, le capot (5) étant indexé en rotation pour imposer le positionnement du rayonnement (R) en face de l'orifice de distribution (45), lors de la mise en place du capot (5) sur le poussoir (4).

## Patentansprüche

1. Ausgabekopf für ein fluides Produkt zum Verbinden mit einer Ausgabeeinrichtung (2), wie einer Pumpe, und einem Behälter (1), um zusammen einen Spender für ein fluides Produkt zu bilden, wobei der Kopf eine Ausgabeöffnung für das fluide Produkt (45) aufweist, durch die das fluide Produkt derart aus dem Kopf austritt, dass es für einen Benutzer zugänglich ist, wobei der Kopf auch eine lösbare Schutzkappe (5) aufweist, die im Lagerzustand die Ausgabeöffnung (45) verdeckt, wobei die Kappe (5) eine Strahlung (R) ausgibt, die dazu geeignet ist, einen möglichen Rest des fluiden Produktes an der Ausgabeöffnung (45) zu bestrahlen, **dadurch gekennzeichnet, dass**
- die Kappe (5) entlang einer axialen Richtung (X) verschiebbar ist, wobei die Ausgabeöffnung (45) quer zu dieser axialen Richtung (X) ausgerichtet ist,
- die Kappe (5) in Bezug auf die Ausgabeöffnung (45) winkelig ausgerichtet ist, um die Positionierung der Strahlung (R) gegenüber der Ausgabeöffnung zu bewirken.

2. Ausgabekopf nach Anspruch 1, wobei die Kappe (5) eine Strahlungsquelle (S) aufweist, die die Strahlung (R) zur Ausgabeöffnung (45) hin ausgibt.

3. Ausgabekopf nach Anspruch 2, wobei die Strahlungsquelle (S) in unmittelbarer Nähe der Ausgabeöffnung (45) angeordnet ist.

4. Ausgabekopf nach Anspruch 2 oder 3, wobei die Strahlungsquelle (S) genau gegenüber der Ausgabeöffnung (45) angeordnet ist.

5. Ausgabekopf nach einem der vorhergehenden Ansprüche, wobei die Ausgabeöffnung (45) durch eine Druckvorrichtung (4) gebildet ist, die entlang einer axialen Richtung (X) hin und her bewegbar ist.

6. Ausgabekopf nach einem der vorhergehenden Ansprüche, wobei die Kappe (45) Auslösemittel (K) für die Strahlung (R) umfasst, die auf das Platzieren der Kappe auf der Ausgabeöffnung (45) reagieren.

7. Ausgabekopf nach Anspruch 6, wobei die Auslösemittel (K) einen Präsenzfühler im Inneren der Kappe aufweisen.

8. Ausgabekopf nach einem der vorhergehenden Ansprüche, wobei die Kappe (45) Verzögerungsmittel (T) umfasst, die dazu geeignet sind, die Strahlung (R) am Ende eines bestimmten Zeitablaufs zu beenden.

9. Ausgabekopf nach einem der vorhergehenden Ansprüche, wobei die Strahlung (R) eine Ultraviolettstrahlung ist, die vorteilhafterweise durch eine Leuchtdiode (LED) ausgegeben wird, die eine Wellenlänge im Bereich von 253 - 254 Nanometern aufweist, um den möglichen Rest des fluiden Produkts an der Ausgabeöffnung (45) zu dekontaminieren oder zu sterilisieren.

10. Spender für ein fluides Produkt, aufweisend:
- einen Behälter für fluides Produkt (1),
- eine Ausgabeeinrichtung (2), wie eine Pumpe, die auf dem Behälter (1) montiert ist, und
- einen Ausgabekopf nach einem der vorhergehenden Ansprüche, der auf der Ausgabeeinrichtung (2) montiert ist,
wobei die Öffnung (45) durch eine Druckvorrichtung (4) gebildet ist, die entlang einer axialen Richtung (X) hin und her bewegbar ist, wobei die Öffnung (45) quer zur axialen Verschiebungsrichtung (X) der Druckvorrichtung (4) ausgerichtet ist, wobei die Kappe (5) an einem festen Teil (3) des Spenders montiert ist und dabei die Druckvorrichtung (4) verdeckt.

11. Spender nach Anspruch 12, wobei die Druckvorrichtung (4) drehfest ist, wobei die Kappe (5) drehindiziert ist, um die Positionierung der Strahlung (R) gegenüber der Ausgabeöffnung (45) zu bewirken, wenn die Kappe (5) auf der Druckvorrichtung (4) platziert wird.

## Claims

1. A fluid dispenser head for associating with a dispenser member (2), such as a pump, and with a reservoir (1), so that together they form a fluid dispenser, the head including a fluid dispenser orifice (45) via which the fluid leaves the head so as to be accessible to a user, the head also including a removable protective cap (5) that masks the dispenser orifice (45) in its storage condition, the cap (5) emitting radiation (R) that is suitable for irradiating any fluid residue at the dispenser orifice (45), the dispenser head being **characterized in that**:
- the cap (5) is movable in an axial direction (X), the dispenser orifice (45) being oriented transversally to the axial direction (X),
- the cap (5) is oriented angularly relative to the dispenser orifice (45) so as to constrain the radiation (R) to be positioned to face the dispenser orifice.

2. A dispenser head according to claim 1, wherein the cap (5) includes a radiation source (S) that emits the radiation (R) towards the dispenser orifice (45).

3. A dispenser head according to claim 2, wherein the radiation source (S) is arranged in the direct proximity of the dispenser orifice (45).

4. A dispenser head according to claim 2 or claim 3, wherein the radiation source (S) is arranged immediately facing the dispenser orifice (45).

5. A dispenser head according to any preceding claim, wherein the dispenser orifice (45) is formed by a pusher (4) that is movable downwards and upwards in an axial direction (X).

6. A dispenser head according to any preceding claim, wherein the cap (45) incorporates trigger means (K) for triggering the radiation (R), which trigger means are sensitive to the cap being put into place over the dispenser orifice (45).

7. A dispenser head according to claim 8, wherein the trigger means (K) comprise a presence detector inside the cap.

8. A dispenser head according to any preceding claim, wherein the cap (45) incorporates timer means (T) that are suitable for interrupting the radiation (R) at the end of a determined period of time.

9. A dispenser head according to any preceding claim, wherein the radiation (R) is an ultra-violet radiation, advantageously emitted by a light-emitting diode (LED), having a wavelength in the range about 253 nm to 254 nm, so as to decontaminate or sterilize any fluid residue at the dispenser orifice (45).

10. A fluid dispenser comprising:
• a fluid reservoir (1);
• a dispenser member (2), such as a pump, mounted on the reservoir (1); and
• a dispenser head according to any preceding claim, mounted on the dispenser member (2);
the orifice (45) being formed by a pusher (4) that is movable downwards and upwards in an axial direction (X), the orifice (45) being oriented transversally to the axial direction of movement (X) of the pusher (4), the cap (5) being mounted on a stationary portion (3) of the dispenser, masking the pusher (4).

11. A dispenser according to claim 12, wherein the pusher (4) is prevented from turning, the cap (5) being indexed in turning so as to constrain the radiation (R) to be positioned to face the dispenser orifice (45), while the cap (5) is being put into place on the pusher (4).
